# EUROPEAN PATENT APPLICATION

(11) **EP 1 238 673 A1**
(43) Date of publication of application: **11.09.2002**
(21) Application number: 00935554.6
(22) Date of filing: 06.06.2000
(51) Int. Cl.: A61K 38/08, A61K 9/16, A23K 1/17

(54) **COLISTIN SULFATE GRANULES**

(30) Priority: 14.12.1999 JP 35426399
(71) Applicant: Asahi Kasei Kabushiki Kaisha, Osaka-shi, Osaka 530-8205 (JP)
(72) Inventor: TSURUGAYA, Moriyuki, Shiraoi-gun, Hokkaido 059-0913 (JP)
(74) Representative: Forstmeyer, Dietmar, Dr. rer. nat., Dipl.-Chem.
(86) International application number: JP0003663
(87) International publication number: WO01043766

(57) **Abstract**

Colistin sulfate granules improved in the stability of potency in feed and exhibiting high solubility can be provided without using expensive carriers or special facilities.

Specifically, said colistin sulfate granules substantially comprise colistin sulfate and have a particle diameters of 150 to 1,500µm, a specific surface area of 40 to 500 cm²/g, a wetting time of 5 minutes or less and a moisture content of 10 % or less.

## Description

### TECHNICAL FIELD

The present invention relates to novel colistin sulfate granules. The colistin sulfate granules of the present invention are colistin sulfate substance substantially consisting of colistin sulfate and useful as a feed additive. The colistin sulfate granules of the present invention exhibit excellent stability of colistin sulfate potency in feed, superior dispersibility, and wettability with water, low generation of dust, and can be used as an additive for feed, drinking water and the like.

### BACKGROUND ART

According to the Minimum Requirements for Antibiotic Products of Japan (1998) colistin is generally obtained by culturing *Bacillus polymyxa var. colistinus* and contains colistin A arid colistin B as major components.

Colistin sulfate is a sulfate of colistin. This is an antibiotic useful not only as a medicine for humans and animals, but also widely used as a feed additive with an objective of growth promotion and improvement of feed efficiency of livestock. Solid colistin sulfate is distributed in the form of fine particles.

Notwithstanding the wide use as a feed additive, colistin sulfate has a problem of unstable potency, particularly when the colistin sulfate is incorporated in pelletized feed prepared by compression forming. Manufacture of a preparation with an objective of improving stability has been studied. For example, Japanese Patent No. 2941585 discloses that the potency stability may be improved by adding α-starch, starch, soybean meal, or a mixture of starch and soybean meal, solidifying, and pulverizing. Japanese Patent Application Laid-open No. 3759/1993 describes coating colistin sulfate with a coating agent such as methylcellulose, paraffin, or gelatin which can increase the potency stability. However, these methods are applied with difficulty to the field of feed additives for which economy is desired because these methods require the addition of α-starch or other various coating agents or special facilities for stabilizing colistin sulfate in the preparation.

In addition, at the present time colistin sulfate is used not only as a feed additive, but also as an additive to drinking water for animals. However, these preparations cannot be used intact for drinking water, because that various. carriers contained in the above colistin sulfate stabilizing preparations are not always easily dissolved in water.

### DISCLOSURE OF THE INVENTION

In the field of feed additives for which economy is required as mentioned above, it is extremely advantageous if the colistin sulfate potency can be stabilized without using expensive various carriers or special facilities. Therefore, improvement of potency stability in feed containing colistin sulfate by any means has been strongly desired.

The present invention has been completed to achieve this technological goal. Specifically, an object of. the present invention is to provide colistin sulfate granules exhibiting high potency 'and improved stability which can be prepared without using expensive carriers or special facilities. Another object of the present invention is to provide colistin sulfate granules exhibiting superior dispersibility and excellent wettability with water, and producing only a slight amount of dust. Still another object of the present invention is to provide a a feed additive obtained using such colistin sulfate granules.

In a vigorous effort to achieve the above objects, the present inventor has prepared colistin sulfate granules substantially comprising colistin sulfate, having a particle diameter of 150-1,500 µm, a specific surface area of'40-500 cm²/g, exhibiting a wetting time of 5 minutes or less and moisture content of 10% or less, and has found that the colistin sulfate granules exhibit good potency stability and superior dispersibility in feed. This finding has led to the completion of the present invention.

Specifically, the present invention provides colistin sulfate granules substantially comprising colistin sulfate, having a specific surface area of 40-500 cm²/g, a wetting time of.5 minutes or less, and a moisture content of 10% or less, the granules substantially comprising particles having a particle diameter of 150-1,500 µm.

In the present invention, colistin sulfate granules 'having a specific surface area of 60-300 cm²/g, a wetting time of 2 minutes or less, and a moisture content of 5% or less, and substantially comprising particles having a particle diameter of 250-850 µm are preferable. In addition, it is desirable that the colistin sulfate granules have a potency of 15,000 units or more per 1 mg.

The present invention further provides a feed additive comprising any one of the above-described colistin sulfate. granules and a carrier for feed additives.

The colistin sulfate granules of the present invention can be conveniently handled without requiring special precautions when added to feed or stored because the colistin sulfate granules do not require any additional stabilization technique for feed. In addition, because special additives need not be added in the manufacturing process, colistin sulfate granules can be provided with excellent economy.

Moreover, as well as the colistin sulfate granules of the present invention can be added to a feed, a solution enable to use as drinking water can be prepared from the colistin sulfate granules of the present invention by a simple dissolution procedure due to superior wettability with water and excellent solubility. Thus, the granules is a colistin sulfate substance with wide utility as both a feed additive and a drinking water additive. Conventional stabilized preparations containing special additives with an objective of stabilizing potency in feed cannot always be used in both feed additives and drinking water additives. Therefore, it is particularly preferable for a stabilized preparation to be provided as a substance substantially consisting of colistin sulfate (hereinafter referred to from time to time as "colistin sulfate substance") like the colistin sulfate'granules of the present invention

Because the colistin sulfate granules of the present invention raise only a slight amount of dust, the product can be added to feed and drinking water with least influence on the health of the workers. Conventional colistin sulfate substance is supplied as fine particles having, for example, a particle diameter of about 2-100 µm, specific surface area of 2,000-5,000 cm²/g, a wetting time of 10 minutes or more, and usually potency of 15,000 units/mg or more. Because this colistin sulfate substance is a fine powder, the products exhibit poor wettability with water and raise considerable dust. In addition, because colistin sulfate is a sulfate, the conventional colistin sulfate substance may irritate the respiratory mucosa and the like of workers, thus involve problems on handling. By contrast, the colistin sulfate granules of the present invention have excellent properties by which the above problems can be overcome.

As the colistin, for example, colistin described in the Minimum Requirements for Antibiotic Products of Japan (1998) can be given, in which a colistin obtained by culturing *Bacillus polymyxa var. colistinus* and containing colistin A and colistin B as major components is described as an example.

Colistin sulfate used in the present invention is a sulfate of colistin. This is an antibiotic useful not only as a medicine for humans and animals, but also widely used as a feed'additive with an objective of growth promotion and improvement of feed efficiency of livestock or the like. Colistin sulfate products commercially available from Asahi Kasei Corporation, Meiji Seika Kaisha, Ltd., and Kaken Pharmaceutical Co., Ltd. can be used in the present invention. These colistin sulfate products are usually known as a mixture of sulfates such as colistin A, colistin B, etc. An isolated, purified colistin thereof may also be used. When the products are used as a medicine, a feed additive, and the like, products satisfying to any one of specifications for colistin substance of the European Pharmacopoeia third edition (1997), the United States Pharmacopeia 23rd edition (1995), the Minimum Requirements for Antibiotic Products of'Japan (1998) , refined grade specifications for feed and feed additive components (Ministerial Ordinance No. 35 of the Ministry of Agriculture, Forestry and Fisheries, 1976, hereinafter referred to from time to time as "Ministerial Ordinance") are preferably used. The colistin sulfate substance or a solution or a liquid thereof may be used. The standards of the colistin sulfate substance in the European Pharmacopoeia (third edition, 1997) , for example, include a potency of 19,000 units/mg or more, pH of 4.0-6.0 in 0.1 g/10 ml aqueous solution, loss on drying of 3.5% or less, and sulfated ash of 1.0% or less. The standards for a liquid substance in the Ministerial Ordinance include a potency of 150 µg (potency)/mg or more, pH of 2.0-5.5, specific gravity of 1.05-1.20, evaporated residue of 40% or less, and ignition residue of 1.25% or less. A fine powdery substance was processed by a granulator under the following conditions, but the substance could not be granulated and remained as fine particles.

### Test conditions

Machine: Roller compactor mini (manufactured by Freund)
Pressure: 100 kg/cm²
Feed rate: 10 rpm
Rolling speed: 3.6 rpm

The lower limit for the particle diameter of the colistin sulfate granules of the present invention is usually 150 µm or more, and preferably 250 µm or more, for example. The upper limit for the particle diameter is usually 1,500 µm or less, and preferably 850 µm or less, for example.

It is particularly preferable that all particles of the colistin sulfate granules of the present invention have the particle diameter of 150-1500 µm, for example. However, it is sufficient that the colistin sulfate granules substantially contain these particles with the diameter range mentioned above, for example, said particles are contained usually in an amount of 80wt% or more, preferably 90wt% or more, and particularly preferably 95wt% or more. The shape of the particles of the colistin sulfate granules is preferably spherical, but not necessarily limited to spherical.

The lower limit of the specific surface area is usually 40 cm²/g or more, and preferably 60 cm²/g or more, for example. The upper limit of the specific surface area is usually 500 cm²/g or less, and preferably 300 cm²/g or less, for example.

The wetting time is usually five minutes or less, and preferably two minutes or less, for example.

The moisture content of the colistin sulfate granules is usually 10% or less, and preferably 5% or less.

The colistin sulfate granules of the present invention usually contain colistin sulfate of 15,000 units/mg (granule weight) or more, and preferably 19,000 units/mg (granule weight) or more, for example. The upper limit of the colistin sulfate content is usually less than 30,000 units/mg (granule weight) , and preferably 25,000 units/mg (granule weight) or less. The particularly preferable range is 20,000-23,000 units/mg (granule weight).

Any method set forth in the European Pharmacopoeia (third edition, 1997), the United States Pharmacopeia (23rd edition, 1995), the Minimum Requirements for Antibiotic Products of Japan (1998), Ministerial Ordinance, or the like can be used for determining the potency of colistin sulfate and unit thereof. For example, the Minimum Requirements for Antibiotic Products of Japan (1998) provides for a bioassay of a cylinder-plate method using agar medium as the test method for the potency. The potency is expressed by the weight (potency) or units as colistin A (free).

As a granulation method for manufacturing the colistin sulfate granules, granulation by a wet method, for example, is preferable. Fluidized bed granulation, spray dry granulation, and rolling granulation can be given. Fluidized bed granulation is a method that fine particles of colistin sulfate substance are granulated while fluidizing in gas phase.. The manner of granulation by spray dry granulation may be considered almost similar to the fluidized bed granulation. Rolling granulation is a method that fine particles of colistin sulfate substance are granulated while rolling by the action of a rolling vessel and stirring blades.

To moisten colistin sulfate granules in these methods, a method of spraying a liquid onto the fluidizing fine particles of colistin sulfate substance, a method of mixing a liquid with fine particles of the colistin sulfate substance and using fine particles of the colistin sulfate thus previously wetted, and the like can be employed. When fluidized bed granulation or spray dry granulation is used, it is possible to manufacture colistin sulfate granules from solution of colistin sulfate substance or the liquid substance specified in the Ministerial Ordinance alone, without using fine particles of colistin sulfate substance. In .this instance, a method employed comprises, for example, producing fine particles of colistin sulfate substance by drying a raw material solution during the initial stage of operation, followed by continuous drying and granulation using the above produced fine particles as cores in the same equipment.

Although there are no specific limitations to the solvent used for moisturizing or dissolving the fine particles, particularly preferable solvents are water, an aqueous solution of colistin sulfate, and the liquid substance described in the Ministerial Ordinance can preferably be used. A nontoxic volatile medium having a boiling point of 150°C or less, such as ethanol or methanol, or a mixture of water and such a volatile medium is also preferable. A volatile medium having a boiling point of 150°C or less, such as benzine or carbon tetrachloride, or a mixture of water and such a volatile medium can also be used.

There are also no specific limitations to the manner of spraying the liquid, the form of nozzles, etc. The spraying direction is also optional inasmuch as the liquid is sprayed toward the fine particles. Spraying upward is preferable in fluidized bed granulation and spray dry granulation.

Granules obtained by the above granulation methods are dried to a moisture content of 10% or less, and preferably 5% or less. After breaking up lumps in the product, if necessary, the granules are sieved to a particle diameter of 150-1,500 µm, and preferably 250-850 µm, for example. The granules preferably have a potency of 15,000-30,000 units/mg, more preferably 19,000-25,000 units/mg, and particularly preferably 20,000-23,000 units/mg, for example.

The granules thus obtained can be used as a colistin sulfate substance substantially consisting of colistin sulfate not only as a medicine for humans and animals, but also as a feed additive with an objective of growth promotion and improvement of feed efficiency of livestock. The granules can also be added to drinking water for animals.

When used as a feed additive, the colistin sulfate granules are mixed with a carrier for feed additives to prepare the feed additive. Any carriers for feed additives may be used without any specific limitations inasmuch as such carriers are not toxic. Examples of the carriers include flour, defatted rice bran, cornstarch, and lactose. This feed additive may be used to form a preparation in which the colistin sulfate is diluted to a potency of 40-100 g (potency) in one kg of the carrier. The feed additive is used by adding to the regular feed for various animals. The livestock to which the feed additive is administered includes chickens, pigs, cattle, and the like. The amount of addition to feed is 2-20 g (potency) per one ton of the feed, for example. The feed may be given to livestock in form such as mashed feed without being formed, or pelleted feed formed by compression forming.

### EXAMPLES

The present invention will now be described in detail by way of examples and test example, which should not be construed as limiting the present invention.

### [Example 1]

A liquid colistin sulfate substance described in the Ministerial Ordinance (the substance concentration: about 175 µg (potency)/mg, manufactured by Asahi Kasei Corporation) was sprayed upward in a fluidized bed granulator ("Splude" manufactured by OKAWARA MFG. CO., LTD.). During a first 30 minute period, the liquid material was sprayed under the conditions of an air velocity of about 1 m/sec., injection temperature of 130-150°C, air exhaustion temperature of 65-80°C, spray air pressure of about 0.15 MPaG, and liquid spray rate of about 5-15 kg/hour, to produce fine particles of colistin sulfate substance which can be used as cores in the vessel. Then, a continuous granulation and drying operation was carried out in the vessel for about five hours under conditions of an injection temperature of about 180°C, air exhaustion temperature of about 60°C, and liquid spray rate of about 20 kg/hour. The resulting granules were continuously discharged by means of a screw and roughly sieved using hot air. Then, only granulated products were taken out. The granulated products thus obtained were placed in a drier maintained at a temperature of 50-60°C to dry to a moisture content of 2.1%, as required, and sieved by a JIS sieve (manufactured by Japan Rikagaku Kikai Co., Ltd., hereinafter the same) to obtain colistin sulfate granules with a particle diameter of 250-480 µm.

### [Example 2]

Granulated products prepared in the same manner as in Example 1, until drying to a moisture content of 2.1%, were sieved by the JIS sieve to obtain colistin sulfate granules with a particle diameter of 840-1410 µm.

### [Example 3]

Granulated products prepared in the same manner as in Example 1, until drying to a moisture content of 2.1%, were sieved by the JIS sieve to obtain colistin sulfate granules with a particle diameter of 149-250 µm.

### [Example 4]

2 kg of colistin sulfate fine particles conforming to the specifications of the European Pharmacopoeia (21,900 units/mg, manufactured by Asahi Kasei Corporation) were placed in a rolling stirrer granulator (a mixing reactor manufactured by Nippon Eirich Co., Ltd.). Water was sprayed onto the fluidizing fine particles at a rate of about 150 ml/min while operating the stirring blade at 900 rpm and rotating the pan at 80 rpm. Granulating conditions were observed from time to time during suspending the spraying operation, stopping the stirring blade or slowing down the rotation to 450 rpm, and stopping the pan or slowing down the rotation to 40 rpm. Spraying was terminated when about 460 ml of water has been sprayed. Then, the particles were processed by rolling for about a further 30 seconds before terminating the granulation operation.

The granulated product was taken out from the pan and spread on a tray. The tray was placed in a drier set at a temperature of 50-60°C to dry the granules to a moisture content of 2.3% while breaking down lumps, if necessary. After breaking down the lumps, the dry product was sieved with the JIS sieve to obtain granules with a particle diameter of 250-840 µm.

### [Example 5]

2 kg of colistin sulfate fine particles conforming to the specifications of the European Pharmacopoeia (21,900 units/mg, manufactured by Asahi Kasei Corporation) were placed in a rolling stirrer granulator (amixing reactor) . An aqueous solution of 20 wt% colistin sulfate was sprayed onto the fluidizing fine particles at a rate of about 150 ml/min while operating the stirring blade at 900 rpm and rotating the pan at 80 rpm. Granulating conditions were observed from time to time by suspending the spraying operation in the same manner as in Example 4. Spraying was terminated when about 610 ml of the aqueous solution of colistin sulfate had been sprayed. Then, the particles were processed by rolling for about a further 30 seconds before terminating the granulation operation. The granulated product was taken out from the pan and spread on a tray. The tray was placed in a drier set at a temperature of 50-60°C to dry the granules to a moisture content of 2.8% while breaking down lumps, if necessary . After breaking down lumps, the dry product was sieved with the JIS sieve to obtain granules with a particle diameter of 250-840 µm.

### [Example 6]

500 g of colistin sulfate fine particles conforming to the specifications of the European Pharmacopoeia (21,900 units/mg, manufactured by Asahi Kasei corporation) were charged into a fluidized bed granulator (a spray granulator manufactured by Powlex Corp.). About 280 ml of water was sprayed onto the fluidizing particles at a rate of about 9.5 ml/min at a suction air temperature of 70°C and a spray air pressure of 1.2 kg/cm². Completion of spraying was judged by observing granulation conditions. After spraying, only hot air was blown to dry the granules until the moisture content became 1.9%. The dry product was taken out and sieved with the JIS sieve to obtain granules with a particle diameter of 177-840 µm.

### [Example 7]

500 g of colistin sulfate fine particles conforming to the specifications of the European Pharmacopoeia (21,900 units/mg, manufactured by Asahi Kasei Corporation) were charged into a fluidized bed granulator (the spray granulator). About 210 ml of a 10 wt% aqueous solution of colistin sulfate was sprayed onto the fluidizing particles at a rate of about 9.5 ml/min at the same suction air temperature and spray air pressure as used in Example 6. Completion of spraying was judged by observing granulation conditions. After spraying, only hot air was blown to dry the granules until the moisture content became 1.8%. The dry product was taken out and sieved by the JIS sieve to obtain granules with a particle diameter of 177-840 µm.

### [Example 8]

1,091 g of granules prepared in Example 1 were homogeneously diluted with 18.9 kg of defatted rice bran to obtain 20 kg of a colistin sulfate feed additive with a colistin sulfate of 4% (potency).

### [Example 9]

2,727 g of granules prepared in Example 1 were homogeneously diluted with 17.3 kg of cornstarch to obtain 20 kg of a colistin sulfate feed additive with a colistin sulfate of 10% (potency).

### [Test Example]

Fine particles o'f colistin sulfate conforming to the specifications of the European Pharmacopoeia (manufactured by Asahi Kasei Corporation) were used as a Comparative Example.

The potency of colistin sulfate was determined by a bioassay using agar medium as described in the European Pharmacopoeia. The potency was indicated by the weight (potency) or units as colistin A (free). Normally, 1 mg (potency) corresponds to 30,000 units.

The specific surface area was measured using a particle size distribution measurement apparatus ("Microtruck" manufactured by Nikkiso Co., Ltd.). The apparatus employs a method of dispersing particle samples in ethanol, measuring the particle size distribution using a laser diffraction scattering method, and determining the specific surface area from the particle size distribution.

### Measurement of wetting time

The wetting time was determined by the following method. Water of a temperature of about 20°C was charged into a 100 ml glass beaker to the mark of 100 ml. The beaker was dipped in a supersonic wave water tank ("Ultrasonic" manufactured by Kagaku Kyoeisha Co., Ltd.). 0.5 g of particle sample was added to the water surface in the beaker while applying supersonic waves to measure the time required for the particles floating on the water surface to sink into the water. This test was repeated three times to determine the average as the wetting time.

### Determination of dissolution time

The dissolution time was determined as follows. A 100 ml glass beaker with a magnetic stirring bar placed therein was charged with water of a temperature of about 20°C to the mark of 100 ml. The beaker was placed on a magnetic stirrer ("Magnester" manufactured by Shibata Scientific Technology, Ltd.) to stir the content at about 300 rpm. 0.5 g of particle sample was added to the surface of water being stirred in the beaker, and the time required for the particles to dissolve into the water was measured. This test was repeated three times to determine the average as the dissolution time.

The potency, specific surface area, wetting time, and dissolution time were determined for the particle samples of Examples 1-7 and Comparative Example. The results are shown in Table 1.

**Table 1**

| | Potency (Units/mg) | Specific surface area (cm²/g) | Wetting time (second) | Dissolution time (second) |
|---|---|---|---|---|
| Example 1 | 22000 | 170 | 21 | 158 |
| Example 2 | 21900 | 50 | 12 | 124 |
| Example 3 | 22000 | 390 | 186 | 231 |
| Example 4 | 21700 | 190 | 33 | 170 |
| Example 5 | 21800 | 180 | 27 | 169 |
| Example 6 | 21400 | 260 | 99 | 189 |
| Example 7 | 21500 | 250 | 104 | 182 |
| Comparative Example | 21900 | 3500 | 610 | 425 |

The results of Table 1 indicate that colistin sulfate fine particles of the Comparative Example required a wetting time of 10 minutes and 10 seconds and a dissolution time of 7 minutes and 5 seconds, whereas colistin sulfate granules of Examples 1-7 exhibited excellent property of a wetting time and dissolution time of 2 minutes or less and 4 minutes or less' respectively.

### Determination of dispersibility

The dispersibility in feed was determined by the following method. Starter feed for calf (manufactured by Nosan Co. , Inc.) was used, 5 kg of feed was put into a godet polyethylene bag (230 mm(length) x 230 mm (width) x 670 mm (height) , polyethylene film thickness: 0.1 mm, manufactured by Noguchi Aluminum Foil Manufacturing Co., Ltd.) and the particle sample was added in an amount to provide a colistin sulfate potency of 40 ppm (about 300 mg of the particle sample) . The bag was filled with air, and the neck was twisted and closed with a rubber band. Then, the contents were vigorously shaken for about one minute to homogeneously disperse the particle samples in the feed. The resulting product is herein called "mashed feed". Five samples of mashed feed, 20 g each, were collected from various parts in the bag to' determine the colistin sulfate potency thereof. Then, the coefficient of variation (CV%) of measured values for the five samples [(Standard deviation of measured values)/(average value) × 100] was determined and taken as the index for dispersibility in feed. A bioassay as described in the Feed Analysis Reference Note (third edition, 1998) was applied to the quantitative determination of colistin sulfate. This method comprises extracting colistin sulfate in samples with a mixture of water, acetone, and hydrochloric acid (51:40:9), and measuring the potency of colistin sulfate using the agar plate stabbing method.

The results of dispersibility determination of Examples 1-3 and Comparative Example are shown in Table 2.

**Table 2**

| | Particle diameter (µm) | Dispersibility index (%) |
|---|---|---|
| Example 1 | 250∼ 840 | 4.5 |
| Example 2 | 840∼1410 | 4.9 |
| Example 3 | 149∼ 250 | 4.1 |
| Comparative Example | 2∼ 100 | 4.2 |

The results shown in Table 2 indicate that the fine particles of the Comparative Example exhibit good dispersibility in feed (the dispersibility index is 4.2%) due to being fine particle, but are handled with difficulty due to generation of a large amount of dust. On the other hand, the colistin sulfate granules of Examples 1-3 not only exhibit good dispersibility in feed (the dispersibility index is 5% or less) , but also have an advantage in low dust generation.

### Stability in feed

The stability in feed was determined by the following method. Mashed feed prepared in the same manner as in the dispersibility experiment was extruded through a pelletizer (manufactured by Joda Ironwork Co., Ltd.). The formed product thus obtained is herein called "pellet feed". The pellet feed was stored at 40°C and 75% RH for four weeks, during which the change in the colistin sulfate potency was determined each week. In the same manner as in the dispersibility experiment, a bioassay described in the Feed Analysis Reference Note (third edition, 1998) was applied to the quantitative determination of colistin sulfate. The stability of colistin sulfate in the pellet feed was indicated by the retained potency percentage (%) to the potency of colistin sulfate in the mashed feed.

The results of the stability determination experiment of Examples 1-3 and Comparative Example are shown in Table 3.

**Table 3**

| | Retained Potency (%) | | | | | |
|---|---|---|---|---|---|---|
| | Mash | Pellets | 1 Week | 2 Weeks | 3 Weeks | 4 Weeks |
| Example 1 | 100 | 98.1 | 94.0 | 85.4 | 82.3 | 81.1 |
| Example 2 | 100 | 98.5 | 96.8 | 87.3 | 85.2 | 86.4 |
| Example 3 | 100 | 97.4 | 88.3 | 83.9 | 78.6 | 76.9 |
| Comparative Example | 100 | 92.5 | 60.1 | 34.5 | 24.4 | 22.3 |

As shown in Table 3, the sample of the Comparative Example exhibits a rapid decrease in potency, whereas the colistin sulfate granules of the present invention were stable.

### INDUSTRIAL APPLICABILITY

The colistin sulfate granules of the present invention can maintain high potency activity, exhibit superior dispersibility and wettability with water, produce only a slight amount of dust, and are stable in feed. Therefore, the colistin sulfate granules are very useful as a medicine for humans and animals, or as a feed additive or an additive being able to add to drinking water.

## Claims

1. Colistin sulfate granules substantially comprising colistin sulfate, having a specific surface area of 40-500 cm²/g, a wetting time of 5 minutes or less, and a moisture content of 10% or less, and substantially comprising particles having a particle diameter of 150-1,500 µm.

2. The colistin sulfate granules according to claim 1, substantially comprising particles having a specific surface area of 60-300 cm²/g, a wetting time of 2 minutes or less, a moisture content of 5% or less, and a particle diameter of 250-850 µm.

3. The colistin sulfate granules according'to claim 1 or claim 2, wherein the colistin sulfate granules have a potency of 15,000 units or more per 1 mg of the colistin sulfate granules.

4. A feed additive comprising the colistin sulfate granules according to any one of claims 1-3 and a carrier for feed.
